# EUROPÄISCHE PATENTSCHRIFT

(11) **EP 1 020 419 B1**
(45) Veröffentlichungstag und Bekanntmachung des Hinweises auf die Patenterteilung: **27.08.2003**
(21) Anmeldenummer: 99121653.2
(22) Anmeldetag: 02.11.1999
(51) Int. Cl.: C07C 7/12, C07C 9/04, B01D 53/02, C01B 3/56

(54) **Verfahren zur Reinigung reduzierend wirkender Gase**
Process for purifying reduction gases
Procédé pour la purification de gaz réducteur

(30) Priorität: 15.01.1999 DE 19901309
(43) Veröffentlichungstag der Anmeldung: 19.07.2000
(73) Patentinhaber: MG Technologies AG, 60325 Frankfurt am Main (DE)
(72) Erfinder: Herden, Hansjörg, Dr., 63110 Rodgau (DE); Weiss, Hans-Jürgen, Dr., 61440 Oberursel (DE); Mayer-Schwinning, Gernot, 61352 Bad Homburg (DE)
(74) Vertreter: Revesz, Veronika

(56) Entgegenhaltungen:
- US-A- 4 101 631
- US-A- 5 620 673

## Beschreibung

Die Erfindung betrifft ein Verfahren zur Abtrennung von Quecksilber, Quecksilberverbindungen, polycyclischen aromatischen Kohlenwasserstoffen (PAK), polyhalogenierten Dibenzodioxinen (PCDD) und polyhalogenierten Dibenzofuranen (PCDF) aus reduzierend wirkenden Gasen, die als Wertstoffe H₂, CO und/oder Kohlenwasserstoffe und als Verunreinigungen H₂S, SO₂, NH₃, HCl, HF, Quecksilber, Quecksilberverbindungen, PAK, PCDD und/oder PCDF enthalten, durch Behandlung der reduzierend wirkenden, verunreinigten Gase mit einem Adsorptionsmittel.

Reduzierend wirkende Gase enthalten als Hauptbestandteile die Wertstoffe H₂, CO und/oder Kohlenwasserstoffe, wobei die reduzierende Wirkung dieser Gase vom Wertstoffgehalt abhängig ist und wobei die Kohlenwasserstoffe insbesondere aus aliphatischen Verbindungen mit 1 bis 5 Kohlenstoffatomen bestehen. Reduzierend wirkende Gase enthalten ferner nur eine geringe Menge oder keinen Sauerstoff. Der maximale Sauerstoffgehalt liegt in der Regel bei ca. 1 %. Reduzierend wirkende Gase kommen entweder als Rohstoffe in der Natur vor oder werden durch technische Prozesse erzeugt. So ist Erdgas wegen seines Gehalts an gasförmigen Kohlenwasserstoffen ein reduzierend wirkendes Gas, und bei der Vergasung und Pyrolyse von Müll, Klärschlamm und organischen Abfällen entsteht ebenfalls ein reduzierend wirkendes Gas, das als Wertstoffe H₂, CO und/oder gasförmige Kohlenwasserstoffe enthält. Die reduzierend wirkenden Gase enthalten außer den Wertstoffen noch CO₂, N₂ und/oder Wasserdampf sowie Verunreinigungen, die als Umweltgifte wirken und die Weiterverarbeitung der reduzierend wirkenden Gase mehr oder weniger stark behindern. Die Verunreinigungen sollten daher nach Möglichkeit bereits vor der Weiterverarbeitung der reduzierend wirkenden Gase abgetrennt werden.

Die reduzierend wirkenden Gase sind häufig mit H₂S, SO₂, NH₃, HCl und HF verunreinigt, wobei diese Verunreinigungen mit bekannten Verfahren abgetrennt werden können, was nicht Gegenstand der vorliegenden Erfindung ist. Die in den reduzierend wirkenden Gasen vorhandenen Verunreinigungen, Quecksilber, Quecksilberverbindungen, PAK, PCDD und PCDF haben eine sehr hohe Toxizität und müssen daher aus den reduzierend wirkenden Gasen vor deren Weiterverarbeitung abgetrennt werden.

Beispielsweise hat Erdgas einen Gehalt an dampfförmigem, metallischem Quecksilber von 0,05 bis 500 µg/Nm³ und die bei der Müllvergasung und Müllpyrolyse anfallenden Gase haben einen Gehalt an dampfförmigem, metallischem Quecksilber von 100 bis 3000 µg/Nm³. Häufig sind die quecksilberhaltigen Gase zusätzlich mit organischen und anorganischen Quecksiberverbindungen verunreinigt. Metallisches Quecksilber ist schon bei Raumtemperatur merklich flüchtig, und daher können Gase erhebliche Mengen an dampfförmigem, metallischem Quecksilber aufnehmen. Beispielsweise enthält mit Quecksilberdampf gesättigte Luft bei 0°C 0,002g Hg(0)/m³, bei 20 °C. 0,014g Hg(0)/m³ und bei 100°C 2,42g Hg(0)/m³. In den bei der Müllvergasung und Müllpyrolyse gebildeten Gasen sind als Verunreinigungen auch PAK, PCDD und PCDF in unterschiedlicher Menge enthalten.

Zur Abtrennung von Quecksilber, Quecksilberverbindungen, PAK, PCDD und PCDF haben sich insbesondere Adsorptionsverfahren bewährt, da sie auch die Entfernung von kleinen Mengen dieser Verunreinigungen ermöglichen. Als Adsorptionsmittel werden insbesondere Aktivkohle, kieselsäurehaltige Adsorbentien oder Zeolithe verwendet. Ferner ist bekannt, daß mit Kupfer, Silber oder Gold beschichtete gebräuchliche Adsorptionsmittel metallisches Quecksilber aus Gasen abtrennen, wobei das Quecksilber in Form der Amalgame gebunden wird. Es ist auch bekannt, daß Salze die Adsorption von metallischem Quecksilber begünstigen, wobei die Salze auf die gebräuchlichen Adsorptionsmittel durch Imprägnierung aufgebracht werden. Ausgehend von diesem allgemein bekannten Fachwissen wurde in der Vergangenheit eine Vielzahl spezieller Adsorptionsverfahren zur Abscheidung von Quecksilber, Quecksilberverbindungen, PAK, PCDD und PCDF vorgeschlagen.

Aus der DE-A 2 227 532 ist ein Verfahren zur Abscheidung von Quecksilber aus Gasen, insbesondere aus elektrolytisch erzeugtem Wasserstoff oder mit Quecksilber verunreinigter Luft, bekannt, bei dem das mit Quecksilber verunreinigte Gas über ein poröses Material geleitet wird, das mit einer Lösung von Salzen des dreiwertigen Eisens getränkt ist. Als poröses Material werden insbesondere Silicagel, Aktivkohle, Tonerde, Silicate oder Metalloxide verwendet. Die Salze des dreiwertigen Eisens sollen das metallische Quecksilber oxidieren und die dabei gebildeten Quecksilbersalze sollen dann in der auf dem porösen Träger befindlichen Tränkungslösung gelöst und damit gebunden werden. Das bekannte Verfahren wird bei einer Temperatur von 0 bis 100°C sowie bei einem Druck > 1 bar durchgeführt.

Aus der DE-C 2 841 565 ist ein Verfahren zur Reinigung von quecksilberhaltigen Gasen bekannt, bei dem das mit Quecksilber verunreinigte Gas mit modifizierten Zeolithen behandelt wird, die in der ionenausgetauschten Form neben den Übergangsmetallen Kupfer, Zink, Cadmium, Mangan und/oder Nickel 0,01 bis 5 Gew.-% Silber enthalten, wobei die Übergangsmetalle und das Silber in reduzierter Form vorliegen. Als Zeolith-Material können sowohl synthetische als auch natürliche Zeolithe verwendet werden. Bei diesem bekannten Verfahren muß das Adsorptionsmittel entweder mit Stickstoff bei 200°C oder mit Wasserstoff bei Temperaturen bis 350°C aktiviert werden, und das quecksilberhaltige Gas muß vor der adsorptiven Entfernung des Quecksilbers getrocknet werden. Das Verfahren arbeitet bei Raumtemperatur und kann zur Abtrennung von Quecksilber aus Wasserstoff, Stickstoff, Luft oder Methan verwendet werden.

Aus der EP-B 0 448 562 ist ein Verfahren zur gleichzeitigen Entfernung von Quecksilber und Wasser aus Erdgas bekannt, bei dem das Erdgas bei einer Temperatur von 0 bis 300°C über einen synthetischen Zeolithen des Typs A geleitet wird, der 0,001 bis 15 Gew.-% elementares Silber oder Gold enthält. Die Regenerierung des Zeolithen erfolgt bei einer Temperatur von 100 bis 600°C.

In der deutschen Patentanmeldung 197 31 639.5 wird ein Verfahren zur Abtrennung von Hg (0) aus reduzierend wirkenden Gasen und Gasgemischen, die außerdem H₂S, NH₃, HCl und/oder Wasserdampf entahlten, durch Adsorption des Hg (0) an einem Adsorptionsmittel vorgeschlagen, das aus einem Gemisch natürlich vorkommender Zeolithe besteht sowie 1 bis 10 Gew.-% Ag, Cu, Sn, Pb, Zn und/oder Cd enthält. Bei diesem Verfahren wird das Hg (0)-haltige Gas oberhalb seines Taupunkts mit dem Adsorptionsmittel bei einer Temperatur von 50 bis 150°C während einer Reaktionszeit von 0,5 bis 10 sec zur Reaktion gebracht. Das Verfahren kann in einem Festbettreaktor, einer zirkulierenden Wirbelschicht oder einem Flugstromreaktor durchgeführt werden.

Die EP-B 0 638 351 schlägt zur Entfernung von Quecksilber aus einem Wasserdampf und SO₂ enthaltenden Abgas ein Verfahren vor, bei dem das Abgas mit einer Temperatur von 20 bis 120°C über einen mit Schwefel oder einer Schwefelverbindung imprägnierten körnigen Zeolithen des Typs X oder Y geleitet wird und das Quecksilber in Form von HgS auf dem Zeolithträger gebunden wird. Als Zeolith wird ein hydrophober, dealuminierter Zeolith verwendet, der im Kristallgitter ein Atomverhältnis Silicium zu Aluminium von 20 zu 1 bis 300 zu 1 aufweist.

US-A-4,101,631 beschreibt ein Verfahren zur Abtrennung von Hg aus Erdgas wobei Erdgas bei einer Temperatur von - 40°C bis 100°C mit Adsorptionsmittel welches Zeolith und Shwefel enthält, behandelt wird.

Schließlich ist bekannt, daß PAK, PCDD und PCDF mehr oder weniger gut an Zeolithen und/oder Aktivkohle adsorbiert werden.

Die Abscheidung von Quecksilber, Quecksilberverbindungen, PAK, PCDD und/oder PCDF aus reduzierend wirkenden Gasen ist nach wie vor problematisch, obwohl bereits verschiedene Verfahren zur Abscheidung dieser Verunreinigungen vorgeschlagen worden sind. Beispielsweise kann das in der deutschen Patentanmeldung 197 31 639.5 vorgeschlagene Reinigungsverfahren technisch erfolgreich eingesetzt werden, aber es hat auch Nachteile, die insbesondere darin bestehen, daß die mit Silber dotierten Zeolithe relativ teuer sind, so daß ihr Einsatz nur dann wirtschaftlich ist, wenn diese Adsorbentien hinreichend oft regeneriert werden können. Die Reinigungsverfahren, die als Adsorptionsmittel Aktivkohle und/oder Zeolithe verwenden sowie bei relativ tiefen Temperaturen arbeiten (ca. 20 bis 110°C) haben den Nachteil, daß die Adsorptionsmittel größere Mengen an Kohlenwasserstoffen adsorbieren, wodurch der Heizwert bzw. der Wertstoffgehalt der reduzierend wirkenden Gase herabgesetzt wird. Schließlich ist es nachteilig, daß die bei hohen Temperaturen erzeugten reduzierend wirkenden Gase zur Abtrennung der Verunreinigungen nach den bekannten Adsorptionsverfahren auf eine Temperatur < 150°C abgekühlt werden müssen.

Der vorliegenden Erfindung liegt die Aufgabe zugrunde, die Nachteile des bekannten Standes der Technik zu vermeiden und ein Adsorptionsverfahren zu schaffen, das ein preiswertes Adsorptionsmittel verwendet, das bei einer höheren Adsorptionstemperatur arbeitet und das die weitgehend quantitative Abtrennung von Quecksilber, Quecksilberverbindungen, PAK, PCDD und/oder PCDF auch in Gegenwart der Verunreinigungen H₂S, SO₂, NH₃, HCl und HF ermöglicht, wobei im Reingas ein Quecksilbergehalt < 50 µg/Nm³, ein PCDD - und PCDF - Gehalt < 0,1 ng TE/Nm³ und ein PAK-Gehalt < 1 µg/Nm³ über einen längeren Zeitraum betriebsicher eingehalten werden muß.

Die der Erfindung zugrunde liegende Aufgabe wird dadurch gelöst, daß die reduzierend wirkenden, verunreinigten Gase bei einer Temperatur von 180 bis 220°C während einer Reaktionszeit von 0,5 bis 10 sec mit einem Adsorptionsmittel behandelt werden, welches Kaolin, Bentonit, einen Zeolith, Ruß, Aktivkohle und/oder Herdofenkoks sowie eine schwefelhaltige Verbindung und/oder Schwefel enthält, daß dann die in den reduzierend wirkenden Gasen suspendierten Feststoffe abgetrennt werden und daß die reduzierend wirkenden Gase anschließend bei 50 bis 75°C und einem pH-Wert < 3 gewaschen werden.

Mit dem erfindungsgemäßen Verfahren ist es möglich, Quecksilber, Quecksilberverbindungen, PAK, PCDD und/oder PCDF nahezu quantitativ und über einen längeren Betriebszeitraum zuverlässig abzutrennen. Im Reingas kann ein Quecksilbergehalt < 50 µg/Nm³ eingehalten werden, der in der Regel sogar < 10 µg/Nm³ ist, und das Reingas hat einen PAK-Gehalt < 1 µg/Nm³ sowie einen PCDD- und PCDF-Gehalt < 0,1 ng TE/Nm³ (TE = Toxizitäts-Äquivalente gemäß NATO-Standard). Das Adsorptionsmittel hat eine Beladungskapazität von 1 bis 10 Gew.-% Hg, wobei das Quecksilber nahezu quantitativ als HgS vorliegt. Die im reduzierend wirkenden Gas enthaltenen Quecksilberverbindungen werden am Adsorptionsmittel ebenfalls abgeschieden und zum überwiegenden Teil in HgS umgewandelt. Bei der Durchführung des erfindungsgemäßen Verfahrens wurde ferner festgestellt, daß weder Kohlenwasserstoffe, noch CO, noch H₂O vom Adsorptionsmittel adsorbiert werden, was auf die erhöhte Adsorptionstemperatur von 180 bis 220°C zurückgeführt wird.

Das erfindungsgemäße Verfahren kann in der Weise durchgeführt werden, daß die reduzierend wirkenden verunreinigten Gase mit dem Adsorptionsmittel in einem Festbettreaktor behandelt werden, wobei das Adsorptionsmittel einen mittleren Teilchendurchmesser d₅₀ von 1 bis 8 mm hat und wobei die Reaktionszeit 1 bis 10 sec und die Gasgeschwindigkeit im Adsorptionsmittel-Festbett 0,05 bis 0,5 m/sec beträgt. Das Adsorptionsmittel ist im Festbettreaktor in Form einer Schüttung angeordnet.

Das erfindungsgemäße Verfahren kann ferner in der Weise durchgeführt werden, daß die reduzierend wirkenden, verunreinigten Gase mit dem Adsorptionsmittel in einer zirkulierenden Wirbelschicht behandelt werden, wobei das Adsorptionsmittel einen mittleren Teilchendurchmesser d₅₀ von 10 bis 100 µm hat und wobei die Reaktionszeit 0,5 bis 5 sec, die Gasgeschwingigkeit 3 bis 8 m/sec und die mittlere Suspensionsdichte der Gas-Feststoff-Suspension 2 bis 10 kg Feststoff/Nm³ beträgt. Die zirkulierende Wirbelschicht ist als Zirkulationssystem gestaltet, das aus einem Reaktor, einem Feststoffabscheider und einer Feststoff-Rückführleitung besteht. Die zirkulierende Wirbelschicht zeichnet sich dadurch aus, daß - im Unterschied zur klassischen Wirbelschicht, bei der eine dichte Phase durch einen deutlichen Dichtesprung von dem darüber befindlichen Gasraum getrennt ist - Verteilungszustände ohne definierte Grenzschicht vorliegen. Ein Dichtesprung zwischen dichter Phase und darüber befindlichem Gasraum ist bei einer zirkulierenden Wirbelschicht nicht existent, jedoch nimmt innerhalb des Reaktors die Feststoffkonzentration von unten nach oben ständig ab. Die Betriebsbedingungen einer zirkulierenden Wirbelschicht sind bekanntlich über die Kennzahlen von Froude und Archimedes bestimmt.

Schließlich kann das erfindungsgemäße Verfahren in der Weise durchgeführt werden, daß die reduzierend wirkenden, verunreinigten Gase mit dem Adsorptionsmittel in einem Flugstromreaktor behandelt werden, wobei das Adsorptionsmittel einen mittleren Teilchendurchmesser d₅₀ von 10 bis 50 µm hat und wobei die Reaktionszeit 0,5 bis 8 sec, die Gasgeschwindigkeit 7 bis 12 m/sec und die mittlere Suspensionsdichte der Gas-Feststoff-Suspension 0,1 bis 3 kg Feststoff/Nm³ beträgt. Im Flugstromreaktor wird das Adsorptionsmittel zunächst in dem zu reinigenden Gasstrom suspendiert, die Gas-Feststoff-Suspension durchläuft dann entsprechend den Verfahrensbedingungen eine Reaktionsstrecke, und danach werden die Fesstoffteilchen aus der Gas-Feststoff-Suspension durch Abscheidevorrichtungen, insbesondere durch einen oder mehrere Zyklone, Schlauchfilter und/oder Elektrofilter abgetrennt.

Nach der Erfindung hat es sich als besonders vorteilhaft erwiesen, wenn als Zeolith ein Gemisch aus natürlich vorkommenden Zeolithen eingesetzt wird, wobei das Gemisch aus natürlich vorkommenden Zeolithen erfindunggemäß aus 10 bis 90 Gew.-% Mordenit, 5 bis 70 Gew.-% Clinoptilolit, 0 bis 5 Gew.-% Montmorillonit und Rest SiO₂ besteht. Ein natürliche Zeolithe enthaltendes Adsorptionsmittel adsorbiert bei der erfindungsgemäßen Adsorptionstemeratur weder CO noch niedere Kohlenwasserstoffe mit 1 bis 8 C-Atomen, und es adsorbiert außerdem kein Wasser.

In weiterer Ausgestaltung der Erfindung ist vorgesehen, daß als schwefelhaltige Verbindung ein Hydrogensulfid, Sulfid, Polysulfid, Dithiocarbonat oder Trithiocarbonat des Natriums, Kaliums oder Calciums oder das Trimercapto-S-triazin eingesetzt wird. Der Schwefel und die schwefelhaltigen Verbindungen können dem Adsorptionsmittel als Pulver zugemischt werden, wobei diese Methode vorteilhaft eingesetzt werden kann, wenn das erfindungsgemäße Verfahren in einer zirkulierenden Wirbelschicht oder in einem Flugstromreaktor durchgeführt wird. Der mittlere Teilchendurchmesser d₅₀ des Schwefels bzw. der schwefelhaltigen Verbindungen beträgt dann 10 bis 50 µm. Es ist ferner möglich, das Adsorptionsmittel mit schwefelhaltigen Verbindungen zu dotieren, was in der Weise geschieht, daß das Adsorptionsmittel mit einer Lösung oder Suspension der schwefelhaltigen Verbindungen getränkt wird und daß das dotierte Adsorptionsmittel anschließend getrocknet wird. Diese Methode kann vorteilhaft angewendet werden, wenn das erfindungsgemäße Verfahren im Festbettreaktor oder in der zirkulierenden Wirbelschicht durchgeführt wird. Schließlich ist es möglich, den Schwefel und die schwefelhaltigen Verbindungen in situ auf das Adsorptionsmittel aufzubringen, indem eine wäßrige Suspension oder Lösung des Schwefels bzw. der schwefelhaltigen Verbindung bei erhöhter Temperatur, z.B. 180 bis 220°C, gemeinsam mit dem Adsorptionsmittel in die zirkulierende Wirbelschicht oder in den Flugstromreaktor eingebracht wird. Hierbei scheidet sich der Schwefel bzw. die schwefelhaltige Verbindung auf dem Adsorptionsmittel in sehr fein verteilter Form ab, und das Lösungsmittel, insbesondere Wasser, verdampft.

Das erfindungsgemäße Verfahren kann besonders vorteilhaft durchgeführt werden, wenn das Adsorptionsmittel aus 1 bis 10 Gew.-% Schwefel und/oder einer schwefelhaltigen Verbindung, 0 bis 10 Gew.-% Ruß, Aktivkohle und/oder Herdofenkoks sowie Rest Kaolin, Bentonit und/oder Zeolith besteht. Dieses Adsorptionsmittel hat eine hohe Adsorptionsleistung, es ist preisgünstig, und es kann sicher gehandhabt werden. Wenn das Adsorptionsmittel einen geringen Gehalt an Ruß, Aktivkohle oder Herdofenkoks aufweist, wird die Adorptionsleistung bezüglich der Verunreinigungen PAK, PCDD und PCDF in vorteilhafter Weise erhöht, wobei der Gehalt des Adsorptionsmittels an aktivem Kohlenstoff die Betriebsicherheit nicht nachteilig beeinflußt.

Alternativ kann das erfindungsgemäße Verfahren besonders vorteilhaft durchgeführt werden, wenn das Adsorptionsmittel aus 1 bis 10 Gew.-% Schwefel und/oder einer schwefelhaltigen Verbindung sowie Rest Gemisch natürlich vorkommender Zeolithe besteht. Dieses Adsorptionsmittel hat sowohl für Quecksilber und dessen Verbindungen als auch für PAK, PCDD und PCDF eine hohe Beladungskapazität.

Alternativ kann das erfindungsgemäße Verfahren ferner besonders vorteilhaft durchgeführt werden, wenn das Adsorptionsmittel aus 1 bis 10 Gew.-% Schwefel und/oder einer schwefelhaltigen Verbindung sowie Rest Ruß, Aktivkohle und/oder Herdofenkoks besteht. Auch dieses Adsorptionsmittel hat sowohl für Quecksilber und dessen Verbindungen als auch für PAK, PCDD und PCDF eine hohe Beladungskapazität,und es gefährdet die Betriebssicherheit nicht, da die zu reinigenden, reduzierend wirkenden Gase keinen Sauerstoff enthalten.

Schließlich hat es sich beim erfindungsgemäßen Verfahren besonders bewährt, wenn die in den reduzierend wirkenden Gasen suspendierten Feststoffe mit einem Schlauchfilter oder einem Elektrofilter oder einem Wäscher oder mittels einer Kombination dieser Vorrichtungen abgetrennt werden, denn mit diesen Prozeßapparaten werden die Feststoffe weitgehend oder quantitativ abgeschieden, so daß die anschließende Gaswäsche entweder nur mit sehr geringen Feststoffmengen belastet ist, oder aber feststofffrei betrieben werden kann.

Nach der Erfindung wird das Verfahren zur Abtrennung von Quecksilber und Quecksilberverbindungen aus Erdgas oder zur Abtrennung von Quecksilber, Quecksilberverbindungen, PAK, PCDD und PCDF aus Gasen, die bei der Vergasung oder Pyrolyse von Müll, Klärschlamm und/oder organischen Abfällen anfallen, vorteilhaft eingesetzt. Erdgas enthält als Hauptbestandteile gasförmige Kohlenwasserstoffe mit 1 bis 4 C-Atomen. Daneben sind im Erdgas H₂, CO₂ und H₂O in unterschiedlicher Menge enthalten. Häufig ist Erdgas durch H₂S und Quecksilber verunreinigt, wobei der Quecksilbergehalt zwischen 0,05 und 500 µg/Nm³ liegt. Bei der Müllvergasung werden die im Müll enthaltenen Kohlenstoffverbindungen in Anwesenheit von Sauerstoff teilweise pyrolysiert, gecrackt und verbrannt. Als Produkt der Müllvergasung entsteht ein Gasgemisch, das in der Regel bis 1 % Sauerstoff, 1 bis 5 % CO, 5 bis 20 % H₂, 10 bis 8000 mg HCl/Nm³, 0,05 bis 1 % H₂S, 500 bis 5000 mg NH₃/Nm³, 100 bis 5000 mg Kohlenwasserstoffe/Nm³ und bis zu 20 % CO₂ enthält (% = Vol.-%). Die durch Müllvergasung erzeugten Gasgemische sind in der Regel mit Wasserdampf gesättigt, und in ihnen liegt das Quecksilber zum weitaus überwiegenden Teil in metallischer Form vor. Die bei der Müllpyrolyse anfallenden Gasgemische enthalten keinen Sauerstoff, und ihr Gehalt an Kohlenwasserstoffen ist höher als bei den durch Müllvergasung erzeugten Gasgemischen, während der Wasserstoffgehalt der durch Müllpyrolyse erzeugten Gasgemische niedriger ist als der Wasserstoffgehalt der durch Müllvergasung erzeugten Gasgemische. Auch in den Gasgemischen der Müllpyrolyse liegt das Quecksilber zum weitaus überwiegenden Teil in metallischer Form vor. In den durch Müllvergasung und Müllpyrolyse erzeugten Gasen sind unterschiedliche Mengen an PAK, PCDD und PCDF enthalten.

Die entsprechend dem erfindungsgemäßen Verfahren gereinigten, reduzierend wirkenden Gase können entweder als Brennstoff oder als chemischer Rohstoff, insbesondere als Reduktonsmittel, verwendet werden. In Abhängigkeit von der weiteren Verwendung der reduzierend wirkenden Gase können die noch vorhandenen Verunreinigungen, insbesondere SO₂, NH₃ und H₂S durch an sich bekannte Reinigungsverfahren abgetrennt werden. Wenn die reduzierend wirkenden Gase als Brennstoff verwendet werden, ist es in der Regel nur noch erforderlich, aus dem Verbrennungsabgas das SO₂ abzutrennen.

Der Gegenstand der Erfindung wird nachfolgend anhand von 2 Ausführungsbeispielen näher erläutert.

### Beispiel 1

Durch Müllvergasung wurde ein Gasgemisch erzeugt, das 17 % H₂, 0,9 % H₂S, 3 % CO, 0,1 % HCl, 1 % O₂ und 78 % N₂ enthielt (% = Vol.-%). Dieses Gasgemisch hatte einen Taupunkt von 70°C und enthielt 600 bis 900 µg Hg (0)/Nm³. Das aus der Vergasung kommende, heiße, staubhaltige Gasgemisch wurde zunächst auf ca. 210°C abgekühlt und danach mit einem Elektrofilter entstaubt. Das entstaubte Gas hatte eine Temperatur von ca. 200°C. Es wurde mit einer Geschwindigkeit von 0,12 m/sec einem Festbettreaktor zugeführt, wobei die mittlere Verweilzeit des Gasgemisches im Festbett 3 sec betrug. Im Festbettreaktor befand sich ein Adsorptionsmittel, das einen mittleren Teilchendurchmesser d₅₀ von 3 mm hatte und das aus 6 Gew.-% Herdofenkoks, 92 Gew.-% eines natürlich vorkommenden Zeolithen und 2 Gew.-% Natriumsulfid bestand. Der natürlich vorkommende Zeolith enthielt 70 Gew.-% Mordenit, 20 Gew.-% Clinoptilolit, 5 Gew.-% Montmorillonit und Rest SiO₂. Das Adsorptionsmittel wurde dadurch hergestellt, daß der natürlich vorkommende Zeolith und der Herdofenkoks zunächst gemischt wurden und daß die Mischung anschließend mit einer wäßrigen Suspension des Natriumsulfids behandelt wurde. Das feuchte Adsorptionsmittel wurde anschließend getrocknet und in den Festbettreaktor als Schüttung eingebracht. Dem Festbettreaktor wurde das quecksilberhaltige Gasgemisch während 250 Stunden zugeführt. Nach dieser Zeit hatte das Adsorptionsmittel einen Quecksilbergehalt von 1,3 Gew.-%. Das den Festbettreaktor verlassende Gas wurde einem Schlauchfilter zugeführt, um restliche Staubteilchen abzutrennen. Anschließend wurde die Temperatur des Gases in einem Wärmeautauscher auf ca. 60°C eingestellt. Das entstaubte und abgekühlte Gas wurde einem Venturi-Wäscher zugeführt, der mit einer wäßrigen Waschflüssigkeit bei einer Temperatur von ca. 60°C betrieben wurde. Die Waschflüssigkeit adsorbierte HCl und hatte einen pH-Wert von ca. 1. Das den Venturi-Wäscher verlassende reduzierend wirkende Gas wurde einem Gaskessel als Brennstoff zugeführt. Das dem Gaskessel zugeführte Gas hatte einen Quecksilbergehalt < 5 µg/Nm³, und in diesem Gas konnten PAK, PCDD und PCDF nicht nachgewiesen werden.

### Beispiel 2

Das gemäß Beispiel 1 durch Müllvergasung erzeugte, staubhaltige Gasgemisch wurde auf ca. 210°C abgekühlt und dann mit einer Geschwindigkeit von 9 m/sec einem Flugstromreaktor zugeführt. Beim Eintritt des Gasgemisches in den Flugstromreaktor wurde im Gasstrom das Adsorptionsmittel suspendiert, das die im Beispiel 1 angegebene Zusammensetzung hatte, das aber einen mittleren Teilchendurchmesser d₅₀ von ca. 20 µm aufwies. Im Flugstromreaktor hatte die Gas-Feststoff-Suspension eine Verweilzeit von ca. 6 sec und eine Suspensionsdichte von 1 kg Feststoff/Nm³. Aus der Gas-Feststoff-Suspension wurde der Feststoff an einem Schlauchfilter abgetrennt, das dem Flugstromreaktor nachgeschaltet war. Der im Schlauchfilter abgeschiedene Feststoff wurde in den Flugstromreaktor zurückgeführt. Der Flugstromreaktor und das Schlauchfilter wurden bei ca. 200 bis 210°C betrieben. Nach einer Betriebszeit von 400 Stunden hatte das Adsorptionsmittel einen Quecksilbergehalt von 2 Gew.-%. Das aus dem Schlauchfilter austretende Gas wurde in einem Wärmeaustauscher auf ca. 60°C abgekühlt und danach einem Venturi-Wäscher zugeführt, der mit einer wäßrigen Waschflüssigkeit bei einer Temperatur von ca. 60°C und einem pH-Wert von ca. 1 betrieben wurde. Das im Gas enthaltene HCl wurde von der Waschflüssigkeit adsorbiert. Das den Venturi-Wäscher verlassende Gas wurde einem Gaskessel als Brennstoff zugeführt und hatte einen Quecksilbergehalt < 5 µg/Nm³. PAK, PCDD und PCDF konnten in dem als Brennstoff verwendeten, reduzierend wirkenden Gas nicht nachgewiesen werden.

Der Begriff Nm³ wird für 1 m³ eines Gases verwendet, das Normalbedingungen, also 1 bar und 0°C, aufweist.

## Patentansprüche

1. Verfahren zur Abtrennung von Quecksilber, Quecksilberverbindungen, polycyclischen aromatischen Kohlenwsserstoffen (PAK), polyhalogenierten Dibenzodioxinen (PCDD) und polyhalogenierten Dibenzofuranen (PCDF) aus reduzierend wirkenden Gasen, die als Wertstoffe H₂, CO und/oder Kohlenwasserstoffe und als Verunreinigungen H₂S, SO₂, NH₃, HCl, HF, Quecksilber, Quecksilberverbindungen, PAK, PCDD und/oder PCDF enthalten, durch Behandlung der reduzierend wirkenden, verunreinigten Gase mit einem Adsorptionsmittel, **dadurch gekennzeichnet, daß** die reduzierend wirkenden, verunreinigten Gase bei einer Temperatur von 180 bis 220°C während einer Reaktionszeit von 0,5 bis 10 sec mit einem Adsorptionsmittel behandelt werden, welches Kaolin, Bentonit, einen Zeolith, Ruß, Aktivkohle und/oder Herdofenkoks sowie eine schwefelhaltige Verbindung und/oder Schwefel enthält, daß dann die in den reduzierend wirkenden Gasen suspendierten Feststoffe abgetrennt werden und daß die reduzierend wirkenden Gase anschließend bei 50 bis 75°C und einem pH-Wert < 3 gewaschen werden.

2. Verfahren nach Anspruch 1, **dadurch gekennzeichnet, daß** die reduzierend wirkenden, verunreinigten Gase mit dem Adsorptionsmittel in einem Festbettreaktor behandelt werden, wobei das Adsorptionsmittel einen mittleren Teilchendurchmesser d₅₀ von 1 bis 8 mm hat und wobei die Reaktionszeit 1 bis 10 sec und die Gasgeschwindigkeit im Adsorptionsmittel-Festbett 0,05 bis 0,5 m/sec beträgt.

3. Verfahren nach Anspruch 1, **dadurch gekennzeichnet, daß** die reduzierend wirkenden, verunreinigten Gase mit dem Adsorptionsmittel in einer zirkulierenden Wirbelschicht behandelt werden, wobei das Adsorptionsmittel einen mittleren Teilchendurchmesser d₅₀ von 10 bis 100 µm hat und wobei die Reaktionszeit 0,5 bis 5 sec, die Gasgeschwindigkeit 3 bis 8 m/sec und die mittlere Suspensionsdichte der Gas-Feststoff-Suspension 2 bis 10 kg Feststoff/Nm³ beträgt.

4. Verfahren nach Anspruch 1, **dadurch gekennzeichnet, daß** die reduzierend wirkenden, verunreinigten Gase mit dem Adsorptionsmittel in einem Flugstromreaktor behandelt werden, wobei das Adsorptionsmittel einen mittleren Teilchendurchmesser d₅₀ von 10 bis 50 µm hat und wobei die Reaktionszeit 0,5 bis 8 sec, die Gasgeschwindigkeit 7 bis 12 m/sec und die mittlere Suspensionsdichte der Gas-Feststoff-Suspension 0,1 bis 3 kg Feststoff/Nm³ beträgt.

5. Verfahren nach den Ansprüchen 1 bis 4, **dadurch gekennzeichnet, daß** als Zeolith ein Gemisch aus natürlich vorkommenden Zeolithen eingesetzt wird.

6. Verfahren nach Anspruch 5, **dadurch gekennzeichnet, daß** das Gemisch aus natürlich vorkommenden Zeolithen aus 10 bis 90 Gew.-% Mordenit, 5 bis 70 Gew.-% Clinoptilolit, 0 bis 5 Gew.-% Montmorillonit und Rest SiO₂ besteht.

7. Verfahren nach den Ansprüchen 1 bis 6, **dadurch gekennzeichnet, daß** als schwefelhaltige Verbindung ein Hydrogensulfid, Sulfid, Polysulfid, Dithiocarbonat oder Trithiocarbonat des Natriums, Kaliums oder Calciums oder das Trimercapto-S-triazin eingesetzt wird.

8. Verfahren nach den Ansprüchen 1 bis 7, **dadurch gekennzeichnet, daß** das Adsorptionsmittel aus 1 bis 10 Gew.-% Schwefel und/oder einer schwefelhaltigen Verbindung, 0 bis 10 Gew.-% Ruß, Aktivkohle und/oder Herdofenkoks sowie Rest Kaolin, Bentonit und/oder Zeolith besteht.

9. Verfahren nach den Ansprüchen 1 bis 7, **dadurch gekennzeichnet, daß** das Adsorptionsmittel aus 1 bis 10 Gew.-% Schwefel und/oder einer schwefelhaltigen Verbindung sowie Rest Gemisch natürlich vorkommender Zeolithe besteht.

10. Verfahren nach den Ansprüchen 1 bis 7, **dadurch gekennzeichnet, daß** das Adsorptionsmittel aus 1 bis 10 Gew.-% Schwefel und/oder einer schwefelhaltigen Verbindung sowie Rest Ruß, Aktivkohle und/oder Herdofenkoks besteht.

11. Verfahren nach den Ansprüchen 1 bis 10, **dadurch gekennzeichnet, daß** die in den reduzierend wirkenden Gasen suspendierten Feststoffe mit einem Schlauchfilter oder einem Elektrofilter oder einem Wäscher oder mittels einer Kombination dieser Vorrichtungen abgetrennt werden.

12. Anwendung des Verfahrens nach den Ansprüchen 1 bis 11 zur Abtrennung von Quecksilber und Quecksilberverbindungen aus Erdgas.

13. Anwendung des Verfahrens nach den Ansprüchen 1 bis 11 zur Abtrennung von Quecksilber, Quecksilberverbindungen, PAK, PCDD und PCDF aus Gasen, die bei der Vergasung oder Pyrolyse von Müll, Klärschlamm und/oder organischen Abfällen anfallen.

## Claims

1. A process for separating off mercury, mercury compounds, polycyclic aromatic hydrocarbons (PAH), polyhalogenated dibenzodioxins (PCDD) and polyhalogenated dibenzofurans (PCDF) from reducing gases which contain the valuable substances H₂, CO and/or hydrocarbons and the impurities H₂S, SO₂, NH₃, HCl, HF, mercury, mercury compounds, PAH, PCDD and/or PCDF, by treating the reducing, contaminated gases with an adsorbent, **characterised in that** the reducing, contaminated gases are treated with an adsorbent which contains kaolin, bentonite, a zeolite, soot, activated carbon and/or blast-furnace coke and also a sulphur-containing compound and/or sulphur at a temperature of 180 to 220°C for a reaction time of 0.5 to 10 seconds, that then the solids suspended in the reducing gases are separated off and that the reducing gases are then scrubbed at 50 to 75°C and a pH value of < 3.

2. A process according to Claim 1, **characterised in that** the reducing, contaminated gases are treated with the adsorbent in a fixed-bed reactor, the adsorbent having an average particle diameter d₅₀ of 1 to 8 mm and the reaction time being 1 to 10 seconds and the gas velocity in the fixed adsorbent bed being 0.05 to 0.5 m/sec.

3. A process according to Claim 1, **characterised in that** the reducing, contaminated gases are treated with the adsorbent in a circulating fluidised bed, the adsorbent having an average particle diameter d₅₀ of 10 to 100 µm and the reaction time being 0.5 to 5 seconds, the gas velocity being 3 to 8 m/sec and the average suspension density of the gas/solids suspension being 2 to 10 kg solids/sm³.

4. A process according to Claim 1, **characterised in that** the reducing, contaminated gases are treated with the adsorbent in an entrained-bed reactor, the adsorbent having an average particle diameter d₅₀ of 10 to 50 µm and the reaction time being 0.5 to 8 seconds, the gas velocity being 7 to 12 m/sec and the average suspension density of the gas/solids suspension being 0.1 to 3 kg solids/sm³.

5. A process according to Claims 1 to 4, **characterised in that** a mixture of naturally occurring zeolites is used as zeolite.

6. A process according to Claim 5, **characterised in that** the mixture of naturally occurring zeolites consists of 10 to 90% by weight mordenite, 5 to 70% by weight clinoptilolite, 0 to 5% by weight montmorillonite and remainder SiO₂.

7. A process according to Claims 1 to 6, **characterised in that** a hydrogen sulphide, sulphide, polysulphide, dithiocarbonate or trithiocarbonate of sodium, potassium or calcium, or trimercapto-S-triazine is used as sulphur-containing compound.

8. A process according to Claims 1 to 7, **characterised in that** the adsorbent consists of 1 to 10% by weight sulphur and/or a sulphur-containing compound, 0 to 10% by weight soot, activated carbon and/or blast-furnace coke and remainder kaolin, bentonite and/or zeolite.

9. A process according to Claims 1 to 7, **characterised in that** the adsorbent consists of 1 to 10% by weight sulphur and/or a sulphur-containing compound and remainder mixture of naturally occurring zeolites.

10. A process according to Claims 1 to 7, **characterised in that** the adsorbent consists of 1 to 10% by weight sulphur and/or a sulphur-containing compound and remainder soot, activated carbon and/or blast-furnace coke.

11. A process according to Claims 1 to 10, **characterised in that** the solids suspended in the reducing gases are separated off using a bag filter or an electrostatic precipitator or a scrubber or by means of a combination of these devices.

12. The application of the process according to Claims 1 to 11 for separating off mercury and mercury compounds from natural gas.

13. The application of the process according to Claims 1 to 11 for separating off mercury, mercury compounds, PAH, PCDD and PCDF from gases which are produced by the gasification or pyrolysis of refuse, clarification sludge and/or organic waste.

## Revendications

1. Procédé de séparation du mercure, de composés du mercure d'hydrocarbures aromatiques polycycliques (HAP), de dibenzodioxines polyhalogénées (DDPC) et dibenzofuranes polyhalogénés (DFPC) dans des gaz réducteurs qui contiennent comme substance de valeur H₂, CO et/ou des hydrocarbures et comme impuretés H₂S, SO₂, NH₃, HCl, HF, du mercure, des composés du mercure, des HAP, des DDPC et/ou des DFPC, par traitement les gaz réducteurs pollués par un agent d'adsorption, **caractérisé en ce que** l'on traite les gaz réducteurs pollués à une température de 180 à 220°C pendant une durée de réaction de 0,5 à 10 s par un agent d'absorption qui contient du kaolin, de la bentonite, un zéolite, de la suie, du carbone actif et/ou du coke de four à sol, ainsi qu'un composé soufré et/ou du soufre, **en ce qu'**ensuite on sépare les matières solides en suspension dans les gaz réducteurs et **en ce que** l'on lave ensuite les gaz réducteurs entre 50 et 75°C et à un pH inférieur à 3.

2. Procédé suivant la revendication 1, **caractérisé en ce que** l'on traite les gaz pollués réducteurs par l'agent d'adsorption dans un réacteur à lit fixe, l'agent d'adsorption ayant un diamètre moyen de particule d₅₀ de 1 à 8 mm et la durée de réaction étant comprise entre 1 et 10 s et la vitesse des gaz dans le lit fixe d'agent d'adsorption étant comprise entre 0,05 à 0,5 m/s.

3. Procédé suivant la revendication 1, **caractérisé en ce que** l'on traite les gaz pollués réducteurs par l'agent d'adsorption dans un lit fluidisé circulant, l'agent d'adsorption ayant une dimension moyenne de particule d₅₀ de 10 à 100 µm et la durée de réaction étant comprise entre 0,5 et 5 s, la vitesse des gaz comprise entre 3 et 8 m/s et la masse volumique moyenne de la suspension gaz-matière solide étant comprise entre 2 et 10 kg de matière solide/m³ pris dans les conditions normales de température et de pression.

4. Procédé suivant la revendication 1, **caractérisé en ce que** l'on traite les gaz pollués réducteurs par l'agent d'absorption dans un réacteur à courant, l'agent d'absorption ayant un diamètre moyen de particule d₅₀ de 10 à 50 µm et la durée de réaction étant comprise entre 0,5 et 8 s, la vitesse des gaz entre 7 et 12 m/s et la masse volumique moyenne de la suspension gaz-matière solide est comprise entre 0,1 et 3 kg de matière solide/m³, pris dans les conditions normales de température de pression.

5. Procédé suivant les revendications 1 à 4, **caractérisé en ce que** l'on utilise comme zéolite un mélange de zéolites d'origine naturelle.

6. Procédé suivant la revendication 5, **caractérisé en ce que** le mélange de zéolites d'origine naturelle est constitué de 10 à 90 % en poids de mordénite, de 5 à 70 % en poids de clinoptilolite, de 0 à 5 % en poids de montmorillonite et le reste étant du SiO₂.

7. Procédé suivant les revendications 1 à 6, **caractérisé en ce que** l'on utilise comme composé soufré un sulfure d'hydrogène, un sulfure, un polysulfure, du diothiocarbonate ou du trithiocarbonate de sodium, de potassium ou de calcium ou la trimercapto-S-triazine.

8. Procédé suivant les revendications 1 à 7, **caractérisé en ce que** l'agent d'adsorption est constitué de 1 à 10 % en poids de soufre et/ou d'un composé soufré, de 0 à 10 % en poids de suie, de carbone actif et/ou de coke de four à sol, ainsi que pour le reste de kaolin, de bentonite et/ou de zéolite.

9. Procédé suivant les revendications 1 à 7, **caractérisé en ce que** l'agent d'adsorption est constitué de 1 à 10 % en poids de soufre et/ou d'un composé soufré, ainsi que pour le reste d'un mélange de zéolites d'origine naturelle.

10. Procédé suivant les revendications 1 à 7, **caractérisé en ce que** l'agent d'adsorption est constitué de 1 à 10 % en poids de soufre et/ou d'un composé soufré et pour le reste de suie, de carbone actif et/ou de coke de four à sol.

11. Procédé suivant l'une des revendications 1 à 10, **caractérisé en ce que** l'on sépare les matières solides en suspension dans les gaz réducteurs par un filtre à manche ou par un électrofiltre ou par un laveur ou au moyen dune combinaison de ces dispositifs.

12. Utilisation du procédé suivant l'une des revendications 1 à 11 pour la séparation du mercure et des composés du mercure dans du gaz naturel.

13. Utilisation du procédé suivant les revendications 1 à 11 pour la séparation du mercure des composés du mercure des HAP, des DDPC et des DFPC dans des gaz qui se produisent lors de la gazéification ou de la pyrolyse des ordures, des boues de clarification et/ou des déchets organiques.
